# EUROPÄISCHE PATENTSCHRIFT

(11) **EP 1 350 476 B1**
(45) Veröffentlichungstag und Bekanntmachung des Hinweises auf die Patenterteilung: **27.04.2005**
(21) Anmeldenummer: 03002810.4
(22) Anmeldetag: 07.02.2003
(51) Int. Cl.: A61B 17/34, A61M 39/06

(54) **Trokarhülse**
Trocar sleeve
Manchon de trocart

(30) Priorität: 02.04.2002 DE 10214552
(43) Veröffentlichungstag der Anmeldung: 08.10.2003
(73) Patentinhaber: Pajunk OHG Besitzverwaltung, 78187 Geisingen (DE)
(72) Erfinder: Pajunk, Horst, 78187 Geisingen (DE); Pajunk, Heinrich, 78187 Geisingen-Kirchen-Hausen (DE)
(74) Vertreter: Manitz, Finsterwald & Partner GbR

(56) Entgegenhaltungen:
- EP-A- 0 513 969
- WO-A-98/09673
- US-A- 5 342 315
- US-A- 6 024 729

## Beschreibung

Die vorliegende Erfindung betrifft eine Trokarhülse mit einem Ventilgehäuse und einem in dem Ventilgehäuse angeordneten Ventil, das einen Einführbereich für ein einzuführendes Instrument besitzt, welcher in distaler Richtung aufeinander zulaufende, als Schrägflächen ausgebildete Wandabschnitte aufweist und in elastisch aneinander anliegenden Dichtlippen endet, wobei die Dichtlippen von einem durch den Einführbereich hindurch geführten Instrument auseinander gedrückt werden.

Um bei der Laparoskopie sowie der minimal invasiven Chirurgie die benötigten Instrumente in das Körperinnere, z. B. die Bauchhöhle einbringen zu können, wird üblicherweise die erforderliche Öffnung in der Bauchdecke durch einen Trokar erzeugt. Auf dem Trokar sitzt eine Trokarhülse, die nach Entfernen des Trokars den Zugangskanal für die verwendeten Instrumente bildet. Um Raum für die Beobachtung bzw. den operativen Eingriff zu schaffen, wird insbesondere bei der minimal invasiven Chirurgie des Bauchraumes Gas unter Druck in die Bauchhöhle eingebracht, um eine Anhebung der Bauchdecke zu erreichen. Damit der auf diese Weise aufgebaute Überdruck aufrechterhalten bleibt, besitzen die Trokarhülsen üblicherweise ein Ventil, das beim Einführen eines Instruments geöffnet wird und in Abwesenheit eines Instruments die Trokarhülse verschließt.

Trokarhülsen der eingangs genannten Art sind in vielfältiger Form bekannt. Problematisch ist jedoch bei den meisten bekannten Trokarhülsen, dass keine ausreichende Dichtigkeit des Ventils gewährleistet ist, insbesondere dass keine ausreichende automatische Schließung des Ventils erfolgt, nachdem ein eingesetztes Instrument aus der Trokarhülse wieder herausgezogen wird. Um die erforderliche Dichtigkeit gewährleisten zu können, werden die Dichtlippen bei bekannten Ventilanordnungen oftmals mit relativ hoher Kraft aneinander gepresst. Dies hat jedoch den Nachteil, dass auf ein in das Körperinnere eingeführtes Instrument eine hohe Klemmkraft wirkt, worunter das Gefühl für feinmechanische Bewegungen des behandelnden Chirurgen leidet. Außerdem ist beim Einführen des Instruments eine relativ hohe Kraft zu überwinden.

Es sind weiterhin Trokarhülsen bekannt, bei denen die Schrägflächen unmittelbar an den Dichtlippen enden, so dass beim Einsetzen eines Instruments in den Einführbereich dieses zwangsläufig auf zumindest einer der Schrägflächen auftrifft. Wird das Instrument nicht exakt mittig in den Einführbereich eingesetzt, so besteht die Gefahr, dass die Schrägfläche, auf die das Instrument auftrifft, durch dieses so stark eingedrückt wird, dass sich das Instrument in der eingedrückten Schrägfläche verhakt und nicht durch das Ventil hindurch in das Körperinnere hineingeführt werden kann.

Es ist eine Aufgabe der vorliegenden Erfindung, eine Trokarhülse der eingangs genannten Art so auszubilden, dass eine sichere Abdichtung der Trokarhülse auch nach Entfernen eines zuvor eingeführten Instrumentes gewährleistet ist. Weiterhin soll verhindert werden, dass beim Einführen des Instrumentes Probleme, beispielsweise in Form von Verhakungen des Instrumentes auftreten können.

Ausgehend von einer Trokarhülse der eingangs genannten Art wird diese Aufgabe erfindungsgemäß dadurch gelöst, dass am distalen Ende des Einführbereiches eine sich quer zur Einführrichtung erstreckende Querwand vorgesehen ist, dass die Schrägflächen jeweils voneinander beabstandet an der Querwand enden, dass die Dichtlippen durch in der Querwand vorgesehene schlitzförmige Öffnungen gebildet sind und dass zwischen den Außenseiten der die Schrägflächen bildenden Wandabschnitte und einer diese Wandabschnitte umfassenden Begrenzungswand taschenförmige Bereiche ausgebildet sind.

Durch die erfindungsgemäße Form des Ventils wird eine besonders sichere Abdichtung der Trokarhülse erzielt. Gleichzeitig wird erreicht, dass beim Einführen eines Instrumentes in den Einführbereich ein Verhaken des Instrumentes praktisch ausgeschlossen ist.

Die erfindungsgemäße verbesserte Abdichtung wird dadurch erreicht, dass an den Außenseiten der die Schrägflächen bildenden Wandabschnitte taschenförmige Bereiche ausgebildet sind, in denen der beispielsweise innerhalb der Bauchhöhle erzeugte Überdruck aufgenommen wird, so dass wiederum ein Druck auf die Außenseiten der Schrägflächen erzeugt wird. Dieser Druck bewirkt zusätzlich zu der Rückstellkraft aufgrund der elastischen Ausbildung der Dichtlippen einen erhöhten Anpressdruck der Dichtlippen aneinander, so dass die Dichtwirkung der Dichtlippen erhöht wird.

Gleichzeitig wird durch die Ausbildung der Dichtlippen in der Querwand und die gleichzeitige Beabstandung der Schrägflächen in Höhe der Querwand voneinander ein im Mittelpunkt des Ventils ausgebildeter freier Bereich der Querwand erzeugt, gegen den ein einzuführendes Instrument anläuft. Es wird somit durch die erfindungsgemäße Ausgestaltung verhindert, dass das Instrument gegen eine der Schrägflächen läuft, was bei einem Eindrücken der Schrägfläche zu einem Verhaken führen könnte. Dadurch dass die Querwand einen mittigen freiliegenden Bereich besitzt, ist gewährleistet, dass das eingeführte Instrument direkt gegen diesen Bereich trifft und unmittelbar die in diesem Bereich aneinander anliegenden Dichtlippen nach innen umbiegt. Ein Verhaken ist bei dieser Ausgestaltung praktisch ausgeschlossen.

Nach einer vorteilhaften Ausführungsform der Erfindung besitzen die Schrägflächen jeweils eine nach innen gewölbte Oberfläche, so dass jeweils die Verbindungskanten zwischen den distalen Enden der Schrägflächen und der Querwand bogenförmig verlaufen. Durch diese Ausgestaltung ist gewährleistet, dass der freiliegende Bereich der Querwand eine ausreichende Größe besitzt, um ein Verhaken des Instrumentes mit den Schrägflächen zuverlässig zu verhindern. Gleichzeitig erzeugt der bogenförmige Querschnitt der Schrägflächen einen optimalen Anpressdruck der Dichtlippen.

Nach einer weiteren vorteilhaften Ausführungsform der Erfindung sind die die innen liegenden Begrenzungsflächen der taschenförmigen Bereiche bildenden Außenseiten der Schrägflächen entsprechend deren Innenflächen nach innen gewölbt. Die taschenförmigen Bereiche besitzen somit eine zur Ventilmitte hin gerichtete konkav gewölbte Fläche, auf die der aus dem Körperinneren wirkende Druck einwirkt. Durch diese spezielle Ausgestaltung ist wiederum ein erhöhter Druck auf die Dichtlippen gewährleistet.

Nach einer weiteren bevorzugten Ausführungsform der Erfindung sind zwischen den Schrägflächen diese verbindende, im Wesentlichen senkrecht zur Querwand stehende, Verbindungsflächen bildende Wandabschnitte vorhanden. Bevorzugt sind dabei die Schrägflächen und/oder die Querwand und/oder die Verbindungsflächen aus elastischem Material, insbesondere aus einem weichelastischen Kunststoff ausgebildet.

Die schlitzförmigen Öffnungen erstrecken sich vorteilhaft jeweils von einer Verbindungsfläche zu einer gegenüberliegenden Verbindungsfläche, um dadurch eine möglichst große Öffnungsfläche beim Einführen eines Instrumentes zu erreichen.

Nach einer weiteren vorteilhaften Ausführungsform der Erfindung verlaufen die schlitzförmigen Öffnungen geradlinig und schneiden sich insbesondere im rechten Winkel. Grundsätzlich sind auch mehrere schlitzförmige Öffnungen möglich, wobei sich diese jeweils bevorzugt unter gleichen Winkeln schneiden. Je nach Anzahl der schlitzförmigen Öffnungen sind somit beispielsweise zwei, drei, vier, sechs, acht oder auch eine sonstige Anzahl von Schrägflächen erreichbar.

Bevorzugt ist jede Schrägfläche mit zumindest einem Versteifungselement versehen. Diese Versteifungselemente können insbesondere als Versteifungsstege ausgebildet sein, die bevorzugt jeweils ausgehend von den proximalen Enden der Schrägflächen in Richtung der distalen Enden der Schrägflächen verlaufen. Durch das Anbringen bzw. das Vorsehen von Verstärkungsstegen wird die Form der Schrägflächen stabilisiert und deren Rückstellkraft erhöht, so dass die Dichtigkeit einer erfindungsgemäßen Trokarhülse weiter verbessert wird. Bevorzugt sind die Versteifungsstege jeweils sich in distaler Richtung verjüngend ausgebildet. Durch diese verjüngende Ausbildung wird zum einen in den Basisabschnitten der Schrägflächen eine höhere Rückstellkraft erzeugt, wobei gleichzeitig aufgrund der verjüngten Enden im Bereich der innen gerichteten Bereiche der Schrägflächen die Klemmkraft etwas reduziert wird, um damit das Einführen eines Instrumentes zu erleichtern bzw. die Bedienbarkeit und Feinfühligkeit für ein eingesetztes Instrument zu verbessern. Grundsätzlich ist es auch möglich, dass an jeder Ventilklappe Verstärkungselemente vorgesehen sind.

Nach einer weiteren bevorzugten Ausführungsform der Erfindung sind die Versteifungselemente jeweils einstückig mit den Schrägflächen ausgebildet und insbesondere an diesen jeweils angeformt. Vorteilhaft sind dabei die Verstärkungselemente aus dem gleichen Material wie die Schrägflächen oder aus einem anderen Material als die Schrägflächen, jedoch ebenfalls elastisch, ausgebildet. Sind die Verstärkungselemente aus dem gleichen Material und insbesondere einstückig mit den Schrägflächen ausgebildet, so kann das gesamte Ventil in einem einfachen Spritzgussverfahren hergestellt werden.

Nach einer weiteren vorteilhaften Ausführungsform der Erfindung sind die Verstärkungselemente jeweils an den innen liegenden Seiten der Schrägflächen vorgesehen. Die innen liegende Anordnung hat den Vorteil, dass beim Einführen eines insbesondere einen größeren Querschnitt aufweisenden Instrumentes dieses beim Einsetzen in den Einführbereich nicht an den Schrägflächen selbst, sondern ausschließlich an den Verstärkungselementen zur Anlage kommt. Dies gilt insbesondere dann, wenn das Instrument einen relativ großen Durchmesser besitzt oder nicht genau mittig in den Einführbereich eingesetzt wird. In diesem Fall wird die Kontaktfläche zwischen dem Instrument und dem Ventil beim Einführen und beim Handhaben des Instrumentes deutlich verringert, so dass auch die Reibungskraft, die zwischen Ventil und Instrument wirkt, verringert wird. Dadurch ist sowohl ein deutlich leichteres Einführen insbesondere von breiten Instrumenten als auch eine bessere Handhabbarkeit der eingesetzten Instrumente möglich.

Grundsätzlich ist es auch möglich, dass die Versteifungselemente an den außen liegenden Seiten der Schrägflächen innerhalb der taschenförmigen Bereiche oder sowohl innen als auch außen liegend vorgesehen sind.

Nach einer weiteren vorteilhaften Ausführungsform der Erfindung ist am proximalen Ende des Einführbereiches ein insbesondere ringförmiger Abschnitt des Ventils ausgebildet. Über den ringförmigen Basisabschnitt ist eine Fixierung des Ventils innerhalb des Ventilgehäuses möglich, wodurch verhindert wird, dass das Ventil beim Einsetzen eines Instrumentes bzw. beim Entnehmen eines Instrumentes aus dem Ventilgehäuse und damit aus der Trokarhülse austreten kann.

Nach einer weiteren vorteilhaften Ausführungsform der Erfindung ist die Begrenzungswand als Teil des Ventilgehäuses oder als separates Teil ausgebildet. Grundsätzlich kann die Begrenzungswand jedoch auch als Teil des Ventils ausgebildet sein. Wesentlich ist, dass die zwischen der Begrenzungswand und den Außenseiten der Schrägflächen gebildeten taschenförmigen Bereiche in proximaler Richtung luftdicht abgeschlossen sind, damit in diesen taschenförmigen Bereichen ein Druck aufgebaut wird, der die Schließkraft des Ventils und damit den Anpressdruck der Dichtlippen aneinander im geschlossenen Zustand des Ventils erhöht.

US 6 024 729 offenbart eine Trokarhülse gemäss dem Oberbegriff des Anspruchs 1.

Weitere vorteilhafte Ausführungsformen der Erfindung sind in den Unteransprüchen angegeben.

Die Erfindung wird nachfolgend anhand eines Ausführungsbeispiels unter Bezugnahme auf die Zeichnung näher erläutert; in dieser zeigen:
- Figur 1: eine Seitenansicht einer erfindungsgemäß ausgebildeten Trokarhülse mit schematisch angedeutetem eingesetzten Instrument,
- Figur 2: eine vereinfachte perspektivische Ansicht eines erfindungsgemäß ausgebildeten Ventils in geschlossenem Zustand,
- Figur 3: eine Unteransicht des Ventils aus Figur 2,
- Figur 4: eine Seitenansicht des Ventils nach Figur 2,
- Figur 5: eine Draufsicht auf den offenen Bereich des Ventils gemäß Figur 2 und
- Figur 6: einen Querschnitt durch das Ventil gemäß der Schnittlinie A - A aus Figur 4 mit gestrichelt dargestellten Instrument.

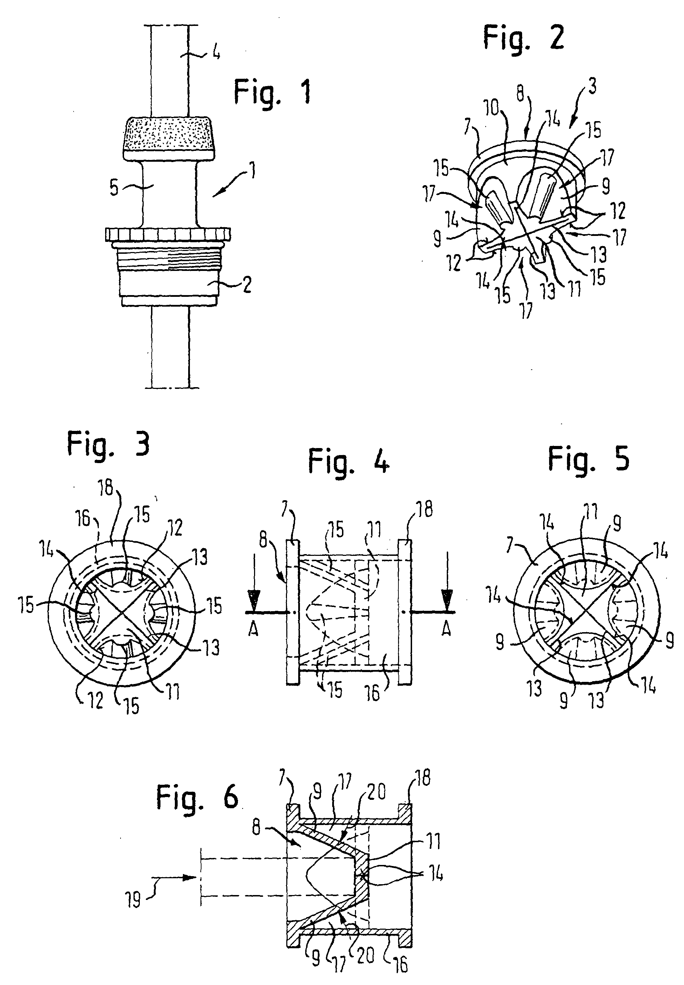

Figur 1 zeigt eine Trokarhülse 1, deren distales Ende als Ventilgehäuse 2 ausgebildet ist. In das offene Ende des Ventilgehäuses 2 ist ein nicht sichtbares Ventil 3 (Figur 2) eingesetzt, das zum Abdichten der Trokarhülse 1 ausgebildet ist. Der Mittelbereich der Trokarhülse 1 ist als Einführkanal ausgebildet, wobei an dem einführseitig gelegenen, proximalen Ende der Trokarhülse 1 eine aus elastischem Kunststoffmaterial ausgebildete Abdichtkappe vorgesehen ist. Diese Abdichtkappe besitzt an ihrer Oberseite eine in der Figur 1 nicht sichtbare, konzentrisch angeordnete Öffnung, durch die ein Instrument 4 in den Einführkanal einführbar ist, wie es in Figur 1 schematisch angedeutet ist. Durch die elastische Abdichtkappe wird dabei die Außenseite des Instrumentes umschlossen und somit abgedichtet, auch wenn das Ventil 3 geöffnet ist. Der Einführkanal ist mit dem Bezugszeichen 5 versehen.

Figur 2 zeigt den grundsätzlichen Aufbau des Ventils 3 in perspektivischer Darstellung, wobei eine in den Figuren 3 bis 6 zusätzlich dargestellte zylinderförmige Begrenzungswand 16 aus Darstellungsgründen weggelassen ist. Das Ventil 3 besitzt an seinem proximalen Ende einen Basisabschnitt 7, der in proximaler Richtung offen ausgebildet ist und das proximale Ende eines Einführbereiches 8 des Ventils 3 bildet. Der Einführbereich 8 ist durch in distaler Richtung aufeinander zulaufende, als Schrägflächen 9 ausgebildete Wandabschnitte gebildet, wobei die Schrägflächen 9 jeweils bogenförmig nach innen gewölbt sind, wie es aus den Figuren 3 und 5 deutlicher ersichtlich ist.

Zwischen den Schrägflächen 9 sind Verbindungsflächen 10 bildende Wandabschnitte vorgesehen, die zusammen mit den Schrägflächen 9 eine vollständige Außenwand des Einführbereiches 8 bilden.

Das distale Ende des Ventils 3 wird durch eine Querwand 11 gebildet, die quer zur Einführrichtung des Instruments 4 verläuft. Die Schrägflächen 9 erstrecken sich vom proximalen Ende bis zu der Querwand 11, wo sie in einer bogenförmigen Kante 12 enden.

Die distalen Enden der Schrägflächen 9 erstrecken sich nicht bis zum Mittelpunkt der Querwand 12, sondern enden entlang der bogenförmigen Kanten 12 beabstandet zueinander. Dadurch wird im Mittelbereich der Querwand 11 ein freier Bereich erzeugt, der sowohl vom distalen Ende her als auch von der proximalen Seite über den Einführbereich 8 her zugänglich ist.

In der Querwand 12 sind zwei geradlinig verlaufende, sich im rechten Winkel schneidende schlitzförmige Öffnungen 13 ausgebildet, die sich im Mittelpunkt der Querwand 11 schneiden und Dichtlippen 14 des Ventils 3 bilden. Aufgrund der gewölbten Oberfläche der Schrägflächen 9 und der Elastizität des Ventilmaterials werden die Dichtlippen 14 aneinander gepresst, so dass ohne eingesetztes Instrument 4 ein Austreten von Gas aus dem Körperinneren durch das Ventil 3 verhindert wird.

Die Abdichtung wird zusätzlich unterstützt durch Versteifungsstege 15, die an der Außenseite der Schrägflächen 9 angeformt sind. Die Versteifungsstege 15 besitzen dabei eine sich in distaler Richtung verjüngende Breite, wie es insbesondere aus Figur 4 zu erkennen ist.

Eine weitere Verstärkung der Abdichtung erfolgt dadurch, dass durch den gewölbten Verlauf der Schrägflächen 9 zwischen deren Außenseite und einer Begrenzungswand 16 taschenförmige Bereiche 17 gebildet werden, in die das in das Körperinnere eingebrachte Gas einströmt, wodurch ein Druck auf die Außenflächen der Schrägflächen 9 erzeugt wird. Auch dies führt dazu, dass die Dichtlippen 14 fest aneinander gepresst werden und somit die Abdichtung des Ventils 3 sicher gewährleistet ist. Die Begrenzungswand 16 ist in Figur 2 zur besseren Darstellung nicht gezeigt, kann jedoch den Figuren 3 bis 6 entnommen werden.

In der in Figur 3 gezeigten Unteransicht des Ventils 3 aus Figur 2 ist besonders gut zu erkennen, dass die die distalen Enden der Schrägflächen 9 bildenden bogenförmigen Kanten 12 voneinander beabstandet sind, so dass die Querwand 11 einen deutlich ausgeprägten freien Bereich zwischen den Schrägflächen 9 besitzt.

Ebenfalls aus Figur 3 ist deutlich zu erkennen, dass die Versteifungsstege 15 unmittelbar an die Außenseiten der Schrägflächen 9 angeformt und mit diesen einstückig ausgebildet sind. Weiterhin ist ein ringförmiger Endabschnitt 18 der Begrenzungswand 16 dargestellt, durch den das Ventil 3 beispielsweise in der Ventilhülse 2 unverschiebbar fixierbar ist.

Der ringförmige Endabschnitt 18 sowie die Begrenzungen 16 sind auch aus der in Figur 4 dargestellten Seitenansicht deutlicher zu erkennen. Außerdem ist aus Figur 4 die sich verjüngende Ausbildung der Versteifungsstege 15 in distaler Richtung gut erkennbar.

Die Draufsicht gemäß Figur 5 zeigt die Ansicht in den Einführbereich 8 hinein. Hier ist deutlich zu erkennen, dass ein von oben eingeführtes Instrument 4 unmittelbar auf die Querwand auftrifft und nicht bzw. nur geringfügig auf die Schrägflächen 9 auftrifft. Dadurch ist ein Eindrücken der Schrägflächen 9 und damit ein Verhaken des Instrumentes 4 praktisch ausgeschlossen, da sich die in der Querwand 11 ausgebildeten Dichtlippen 14 direkt nach Auftreffen des Instrumentes 4 nach unten öffnen und somit ein ungehindertes Eintreten des Instrumentes 4 in das Körperinnere gewährleisten. Dies ist auch aus der Schnittdarstellung gemäß Figur 6 erkennbar, in der schematisch ein gestrichelt dargestelltes Instrument 4 gezeigt ist, das entlang eines Pfeils 19 in den Einführbereich 8 des Ventils 3 eingeführt wird und unmittelbar an der Querwand 11 zur Anlage kommt.

Aus dieser Darstellung sind sehr deutlich die taschenförmigen Bereiche 17 zu erkennen, in denen aufgrund des in dem Körperinneren vorhandenen Drucks ebenfalls ein Überdruck vorhanden ist, der entsprechend Pfeilen 20 eine Kraft auf die Außenflächen der Schrägfläche 9 bewirkt, so dass diese und damit auch die Dichtlippen 14 zusammengedrückt werden, wodurch die Abdichtung des Ventils 3 verbessert wird.

### Bezugszeichenliste

- 1: Trokarhülse
- 2: Ventilgehäuse
- 3: Ventil
- 4: Instrument
- 5: Einführkanal
- 7: Basisabschnitt
- 8: Einführbereich
- 9: Schrägflächen
- 10: Verbindungsflächen
- 11: Querwand
- 12: bogenförmige Kanten
- 13: schlitzförmige Öffnungen
- 14: Dichtlippen
- 15: Versteifungsstege
- 16: Begrenzungswand
- 17: taschenförmige Bereiche
- 18: Endabschnitt
- 19: Pfeil
- 20: Pfeil

## Patentansprüche

1. Trokarhülse mit einem Ventilgehäuse (2) und mit einem in dem Ventilgehäuse (2) angeordneten Ventil (3), das einen Einführbereich (8) für ein einzuführendes Instrument (4) besitzt, welcher in distaler Richtung aufeinander zulaufende, als Schrägflächen (9) ausgebildete Wandabschnitte aufweist und in elastisch aneinander anliegenden Dichtlippen (14) endet, wobei die Dichtlippen (14) von einem durch den Einführbereich (8) hindurch geführten Instrument (4) auseinander gedrückt werden und wobei zwischen den Außenseiten der die Schrägflächen (9) bildenden Wandabschnitte und einer diese Wandabschnitte umfassenden Begrenzungswand (16) taschenförmige Bereiche (17) ausgebildet sind.
**dadurch gekennzeichnet,**
**dass** am distalen Ende des Einführbereichs (8) eine sich quer zur Einführrichtung (19) erstreckende Querwand (11) vorgesehen ist, dass die Schrägflächen (9) jeweils voneinander beabstandet an der Querwand (11) enden, dass die Dichtlippen (14) durch in der Querwand (11) vorgesehene schlitzförmige Öffnungen (13) gebildet sind.

2. Trokarhülse nach Anspruch 1,
**dadurch gekennzeichnet,**
**dass** die Schrägflächen (9) jeweils eine nach innen gewölbte Oberfläche besitzen, so dass jeweils die Verbindungskanten (12) zwischen den distalen Enden der Schrägflächen (9) und der Querwand (11) bogenförmig verlaufen.

3. Trokarhülse nach Anspruch 2,
**dadurch gekennzeichnet,**
**dass** die die innen liegenden Begrenzungsflächen der taschenförmigen Bereiche (17) bildenden Außenseiten der Schrägflächen (9) entsprechend deren Innenflächen nach innen gewölbt sind.

4. Trokarhülse nach einem der vorhergehenden Ansprüche,
**dadurch gekennzeichnet,**
**dass** zwischen den Schrägflächen (9) diese verbindende, im Wesentlichen senkrecht zur Querwand (11) stehende, Verbindungsflächen (10) bildende Wandabschnitte vorhanden sind.

5. Trokarhülse nach einem der vorhergehenden Ansprüche oder nach Anspruch 4,
**dadurch gekennzeichnet,**
**dass** die Schrägflächen (9) und/oder die Querwand (11) und/oder die Verbindungsflächen (10) aus elastischem Material, insbesondere aus Kunststoff, ausgebildet ist.

6. Trokarhülse nach einem der Ansprüche 4 oder 5,
**dadurch gekennzeichnet,**
**dass** sich die schlitzförmigen Öffnungen (13) jeweils von einer Verbindungsfläche (10) zu einer gegenüberliegenden Verbindungsfläche (10) erstrecken.

7. Trokarhülse nach einem der vorhergehenden Ansprüche,
**dadurch gekennzeichnet,**
**dass** sich die schlitzförmigen Öffnungen (13) im Mittelpunkt der Querwand (11) schneiden.

8. Trokarhülse nach einem der vorhergehenden Ansprüche,
**dadurch gekennzeichnet,**
**dass** die schlitzförmigen Öffnungen (13) geradlinig verlaufen und sich insbesondere im rechten Winkel schneiden.

9. Trokarhülse nach einem der vorhergehenden Ansprüche,
**dadurch gekennzeichnet,**
**dass** jede Schrägfläche (9) mit zumindest einem Versteifungselement (15) versehen ist.

10. Trokarhülse nach Anspruch 9,
**dadurch gekennzeichnet,**
**dass** die Versteifungselemente als Versteifungsstege (15) ausgebildet sind.

11. Trokarhülse nach Anspruch 10,
**dadurch gekennzeichnet,**
**dass** die Versteifungsstege (15) jeweils ausgehend von den proximalen Enden der Schrägflächen (9) in Richtung der distalen Enden der Schrägflächen (9) verlaufen.

12. Trokarhülse nach Anspruch 10 oder 11,
**dadurch gekennzeichnet,**
**dass** die Versteifungsstege (15) jeweils sich in distaler Richtung verjüngend ausgebildet sind.

13. Trokarhülse nach einem der Ansprüche 9 bis 12,
**dadurch gekennzeichnet,**
**dass** die Versteifungselemente (15) jeweils einstückig mit den Schrägflächen (9) ausgebildet sind.

14. Trokarhülse nach einem der Ansprüche 9 bis 13,
**dadurch gekennzeichnet,**
**dass** die Versteifungselemente (15) aus dem gleichen Material wie die Schrägflächen (9) oder aus einem anderen Material als die Schrägflächen (9), jedoch ebenfalls elastisch, ausgebildet sind.

15. Trokarhülse nach einem der Ansprüche 9 bis 14,
**dadurch gekennzeichnet,**
**dass** die Versteifungselemente (15) an den innen liegenden Seiten der Schrägflächen (9) vorgesehen sind.

16. Trokarhülse nach einem der Ansprüche 9 bis 15,
**dadurch gekennzeichnet,**
**dass** die Versteifungselemente (15) an den außen liegenden Seiten der Schrägflächen (9) innerhalb der taschenförmigen Bereiche (17) vorgesehen sind.

17. Trokarhülse nach einem der vorhergehenden Ansprüche,
**dadurch gekennzeichnet,**
**dass** am proximalen Ende des Einführbereichs (8) ein insbesondere ringförmiger Basisabschnitt (7) des Ventils (3) ausgebildet ist.

18. Trokarhülse nach einem der vorhergehenden Ansprüche,
**dadurch gekennzeichnet,**
**dass** die Begrenzungswand (16) als Teil des Ventilgehäuses (2) oder als separates Teil ausgebildet ist.

19. Trokarhülse nach einem der Anspruche 1 bis 17,
**dadurch gekennzeichnet,**
**dass** die Begrenzungswand (16) als Teil des Ventils (3) ausgebildet ist.

20. Trokarhülse nach einem der vorhergehenden Ansprüche,
**dadurch gekennzeichnet,**
**dass** die Begrenzungswand (16) luftdicht mit dem proximalen Ende des Ventils (3) verbunden ist.

21. Trokarhülse nach einem der vorhergehenden Ansprüche,
**dadurch gekennzeichnet,**
**dass** die Begrenzungswand (16) luftdicht mit dem Basisabschnitt (7) des Ventils (3) verbunden ist.

22. Trokarhülse nach einem der vorhergehenden Ansprüche,
**dadurch gekennzeichnet,**
**dass** die Begrenzungswand (16) im Bereich der proximalen Enden der Schrägflächen (9) luftdicht mit dem Ventil (3) verbunden ist.

23. Trokarhülse nach einem der vorhergehenden Ansprüche,
**dadurch gekennzeichnet,**
**dass** das Ventil (3) einstückig, insbesondere als einstückiges Kunststoffteil ausgebildet ist.

## Claims

1. A trocar sleeve comprising a valve housing (2) and a valve (3) which is arranged in the valve housing (2) and has an insertion region (8) for an instrument (4) to be introduced, said insertion region having wall sections, which converge towards one another in the distal direction and are made as sloping surfaces (9), said insertion region ending in sealing lips (14) which contact one another elastically, with the sealing lips (14) being pressed apart by an instrument (4) guided through the insertion region (8) and with pocket-like regions (17) being formed between the outer sides of the wall sections forming the sloping surfaces (9) and a boundary wall (16) surrounding these wall sections,
**characterised in that**
a transverse wall (11) extending transversely to the insertion direction (19) is provided at the distal end of the insertion region (8); **in that** the sloping surfaces (9) each end spaced apart from one another at the transverse wall (11); and **in that** the sealing lips (14) are formed by slot-like openings (13) provided in the transverse wall (11).

2. A trocar sleeve in accordance with claim 1, **characterised in that** the sloping surfaces (9) each have an inwardly arched surface such that the respective connection edges (12) extend in arcuate form between the distal ends of the sloping surfaces (9) and the transverse wall (11).

3. A trocar sleeve in accordance with claim 2, **characterised in that** the outer sides of the sloping surfaces (9) forming the inwardly disposed boundary surfaces of the pocket-like regions (17) are inwardly arched in accordance with their inner surfaces.

4. A trocar sleeve in accordance with any one of the preceding claims, **characterised in that** wall sections which stand essentially perpendicular to the transverse wall (11) are present between the sloping surfaces (9), connect them, and form connection surfaces (10).

5. A trocar sleeve in accordance with any one of the preceding claims or in accordance with claim 4, **characterised in that** the sloping surfaces (9) and/or the transverse wall (11) and/or the connection surfaces (10) are made of resilient material, in particular of plastic.

6. A trocar sleeve in accordance with one of the claims 4 or 5, **characterised in that** the slot-like openings (13) each extend from one connection surface (10) to an oppositely disposed connection surface (10).

7. A trocar sleeve in accordance with any one of the preceding claims, **characterised in that** the slot-shaped openings (13) intersect at the centre of the transverse wall (11).

8. A trocar sleeve in accordance with any one of the preceding claims, **characterised in that** the slot-like openings (13) extend in a straight line and in particular intersect at a right angle.

9. A trocar sleeve in accordance with any one of the preceding claims, **characterised in that** each sloping surface (9) is provided with at least one stiffening element (15).

10. A trocar sleeve in accordance with claim 9, **characterised in that** the stiffening elements are made as stiffening webs (15).

11. A trocar sleeve in accordance with claim 10, **characterised in that** the stiffening webs (15) extend in the direction of the distal ends of the sloping surfaces (9), in each case starting from the proximal ends of the sloping surfaces (9).

12. A trocar sleeve in accordance with claim 10 or claim 11, **characterised in that** the stiffening webs (15) each taper in the distal direction.

13. A trocar sleeve in accordance with any one of the claims 9 to 12, **characterised in that** the stiffening elements (15) are each made in one piece with the sloping surfaces (9).

14. A trocar sleeve in accordance with any one of the claims 9 to 13, **characterised in that** the stiffening elements (15) are made of the same material as the sloping surfaces (9) or of a different material to the sloping surfaces (9), but likewise resiliently.

15. A trocar sleeve in accordance with any one of the claims 9 to 14, **characterised in that** the stiffening elements (15) are provided at the inwardly disposed sides of the sloping surfaces (9).

16. A trocar sleeve in accordance with any one of the claims 9 to 15, **characterised in that** the stiffening elements (15) are provided at the outwardly disposed sides of the sloping surfaces (9) inside the pocket like regions (17).

17. A trocar sleeve in accordance with any one of the preceding claims, **characterised in that** a base section (7), in particular a ring-shaped base section, of the valve (3) is formed at the proximal end of the insertion region (8).

18. A trocar sleeve in accordance with any one of the preceding claims, **characterised in that** the boundary wall (16) is made as part of the valve housing (2) or as a separate part.

19. A trocar sleeve in accordance with any one of the claims 1 to 17, **characterised in that** the boundary wall (16) is made as part of the valve (3).

20. A trocar sleeve in accordance with any one of the preceding claims, **characterised in that** the boundary wall (16) is connected to the proximal end of the valve (3) in an airtight manner.

21. A trocar sleeve in accordance with any one of the preceding claims, **characterised in that** the boundary wall (16) is connected to the base section (7) of the valve (3) in an airtight manner.

22. A trocar sleeve in accordance with any one of the preceding claims, **characterised in that** the boundary wall (16) is connected to the valve (3) in an airtight manner in the region of the proximal ends of the sloping surfaces (9).

23. A trocar sleeve in accordance with any one of the preceding claims, **characterised in that** the valve (3) is made as one piece, in particular as a one-piece plastic part.

## Revendications

1. Douille de trocart comportant un corps (2) de valve et une valve (3) disposée dans le corps (2) de valve, laquelle possède une zone d'insertion (8) pour un instrument (4) à insérer, laquelle comporte des tronçons de paroi agencés sous la forme de surfaces obliques (9) qui convergent l'une vers l'autre dans la direction distale, et se termine dans des lèvres d'étanchéité (14) s'appliquant de façon élastique l'une contre l'autre, les lèvres d'étanchéité (14) étant écartées l'une de l'autre par un instrument (4) engagé dans la zone d'insertion (8), et des zones en forme de poches (17) étant agencées entre les faces extérieures des tronçons de paroi formant les surfaces obliques (9) et une paroi de délimitation (16) entourant ces tronçons de paroi,
**caractérisée en ce que**
une paroi transversale (11) s'étendant transversalement au sens d'insertion (19) est prévue sur l'extrémité distale de la zone d'insertion (8), **en ce que** les surfaces obliques (9) se terminent respectivement au niveau de la paroi transversale (11) en étant écartées l'une de l'autre, et **en ce que** les lèvres d'étanchéité (14) sont formées par des ouvertures (13) en forme de fentes prévues dans la paroi transversale (11).

2. Douille de trocart selon la revendication 1,
**caractérisée en ce que**
les surfaces obliques (9) possèdent chacune une surface incurvée vers l'intérieur, de sorte que les bords de liaison (12) s'étendent respectivement de façon cintrée entre les extrémités distales des surfaces obliques (9) et la paroi transversale (11).

3. Douille de trocart selon la revendication 2,
**caractérisée en ce que**
les faces extérieures des surfaces obliques (9), qui forment les surfaces de délimitation situées à l'intérieur des zones en forme de poches (17), sont incurvées vers l'intérieur de façon correspondante à leurs surfaces intérieures.

4. Douille de trocart selon l'une quelconque des revendications précédentes,
**caractérisée en ce que**,
entre les surfaces obliques (9), il est prévu des tronçons de paroi formant des surfaces de liaison (10) reliant ces surfaces entre elles, qui sont pour l'essentiel positionnées perpendiculairement à la paroi transversale (11).

5. Douille de trocart selon l'une quelconque des revendications précédentes ou selon la revendication 4,
**caractérisée en ce que**
les surfaces obliques (9) et/ou la paroi transversale (11) et/ou les surfaces de liaison (10) sont réalisées en un matériau élastique, notamment en matière plastique.

6. Douille de trocart selon l'une des revendications 4 ou 5,
**caractérisée en ce que**
les ouvertures (13) en forme de fentes s'étendent respectivement d'une surface de liaison (10) vers une surface de liaison (10) située en face.

7. Douille de trocart selon l'une quelconque des revendications précédentes,
**caractérisée en ce que**
les ouvertures (13) en forme de fentes se coupent au centre de la paroi transversale (11).

8. Douille de trocart selon l'une quelconque des revendications précédentes,
**caractérisée en ce que**
les ouvertures (13) en forme de fentes s'étendent en ligne droite et se coupent notamment à angle droit.

9. Douille de trocart selon l'une quelconque des revendications précédentes,
**caractérisée en ce que**
chaque surface oblique (9) est pourvue d'au moins un élément de raidissement (15).

10. Douille de trocart selon la revendication 9,
**caractérisée en ce que**
les éléments de raidissement sont agencés sous la forme d'entretoises de raidissement (15).

11. Douille de trocart selon la revendication 10,
**caractérisée en ce que**
les entretoises de raidissement (15) s'étendent respectivement à partir des extrémités proximales des surfaces obliques (9) vers les extrémités distales des surfaces obliques (9).

12. Douille de trocart selon la revendication 10 ou 11,
**caractérisée en ce que**
les entretoises de raidissement (15) sont agencées en s'effilant respectivement dans la direction distale.

13. Douille de trocart selon l'une quelconque des revendications 9 à 12,
**caractérisée en ce que**
les éléments de raidissement (15) sont respectivement réalisés d'un seul tenant avec les surfaces obliques (9).

14. Douille de trocart selon l'une quelconque des revendications 9 à 13,
**caractérisée en ce que**
les éléments de raidissement (15) sont constitués du même matériau que les surfaces obliques (9) ou d'un autre matériau que les surfaces obliques (9), mais sont également agencés de façon élastique.

15. Douille de trocart selon l'une quelconque des revendications 9 à 14,
**caractérisée en ce que**
les éléments de raidissement (15) sont prévus sur les faces intérieures des surfaces obliques (9).

16. Douille de trocart selon l'une quelconque des revendications 9 à 15,
**caractérisée en ce que**
les éléments de raidissement (15) sont prévus sur les faces extérieures des surfaces obliques (9) à l'intérieur des zones en forme de poches (17).

17. Douille de trocart selon l'une quelconque des revendications précédentes,
**caractérisée en ce que**
un tronçon de base (7) de la valve (3), notamment de forme annulaire, est agencé sur l'extrémité proximale de la zone d'insertion (8).

18. Douille de trocart selon l'une quelconque des revendications précédentes,
**caractérisée en ce que**
la paroi de délimitation (16) est agencée en tant que partie du corps (2) de valve ou en tant que partie séparée.

19. Douille de trocart selon l'une quelconque des revendications 1 à 17,
**caractérisée en ce que**
la paroi de délimitation (16) est agencée en tant que partie de la valve (3).

20. Douille de trocart selon l'une quelconque des revendications précédentes,
**caractérisée en ce que**
la paroi de délimitation (16) est reliée de façon étanche à l'air à l'extrémité proximale de la valve (3).

21. Douille de trocart selon l'une quelconque des revendications précédentes,
**caractérisée en ce que**
la paroi de délimitation (16) est reliée de façon étanche à l'air au tronçon de base (7) de la valve (3).

22. Douille de trocart selon l'une quelconque des revendications précédentes,
**caractérisée en ce que**,
dans la zone des extrémités proximales des surfaces obliques (9), la paroi de délimitation (16) est reliée de façon étanche à l'air à la valve (3).

23. Douille de trocart selon l'une quelconque des revendications précédentes,
**caractérisée en ce que**
la valve (3) est réalisée d'un seul tenant, notamment sous la forme d'une pièce en matière plastique d'un seul tenant.
